# EUROPEAN PATENT APPLICATION

(11) **EP 3 018 113 A1**
(43) Date of publication of application: **11.05.2016**
(21) Application number: 14192088.4
(22) Date of filing: 06.11.2014
(51) Int. Cl.: C07C 1/24, C07C 1/06, C07C 1/04

(54) **Process and apparatus for the production of ethylene from carbon monoxide and hydrogen**

(71) Applicant: BP p.l.c., London SW1Y 4PD (GB)
(72) Inventor: The designation of the inventor has not yet been filed
(74) Representative: Hill, Simon Stephen

(57) **Abstract**

A process for the conversion of carbon monoxide and hydrogen to ethylene, said process comprising the following steps:
i) introducing carbon monoxide, hydrogen and methyl acetate into an alcohol synthesis unit to produce a first product stream comprising methanol and ethanol;
ii) separating a methanol and ethanol rich stream and a carbon monoxide and hydrogen rich stream from the first product stream;
iii) introducing at least part of the methanol and ethanol rich stream into a dehydration reactor to produce a second product stream comprising ethylene and dimethyl ether;
iv) separating an ethylene product stream and a dimethyl ether product stream from the second product stream; and
v) introducing carbon monoxide, hydrogen and at least part of the dimethyl ether product stream into a carbonylation reactor to produce a third product stream comprising methyl acetate;
wherein at least a part of the methyl acetate introduced into the alcohol synthesis unit in step i) is recycled from the third product stream produced from the carbonylation reactor in step v); and
wherein the alcohol synthesis unit of step i) comprises a catalyst which is effective to perform hydrogenolysis of methyl acetate and a catalyst which is effective to produce methanol from carbon monoxide and hydrogen, preferably in the form of synthesis gas; and
wherein the carbonylation reactor of step v) comprises a zeolite catalyst effective for said carbonylation reaction.

## Description

This invention relates to a process and an apparatus for the production of ethylene from carbon monoxide and hydrogen, preferably derived from a synthesis gas feedstream. In particular, the invention relates to an improved process and apparatus configuration which provides energy efficiency, whilst maintaining high ethylene product yield by recycling of by-products. The present invention benefits from the integration of multiple reactions involved at different stages in the conversion of carbon monoxide and hydrogen to ethylene, so as to provide a conversion process of good selectivity which makes efficient use of a few reactors.

Ethylene is an important commodity chemical and monomer which has traditionally been produced industrially by the steam or catalytic cracking of hydrocarbons derived from crude oil. However, due to the potential uncertainties in the availability, security of supply, and price of crude oil, there becomes an increasing need to find alternative economically viable methods of making this product. By virtue of its ready availability from the fermentation of biomass and synthesis gas based technologies, ethanol is emerging as an important potential feedstock from which ethylene can be made in the future.

Synthesis gas refers to a combination of H₂ and carbon oxides, typically produced in a synthesis gas plant from a carbonaceous feedstock, such as natural gas, petroleum liquids, biomass, coal, recycled plastics, municipal wastes, or any organic material and mixtures thereof. Thus, ethanol and ethanol derivatives may provide a non-petroleum based route for the production of ethylene.

Generally, the production of ethanol from synthesis gas takes place via three process stages: synthesis gas preparation, ethanol synthesis, and ethanol purification. In the synthesis gas preparation step, an additional stage may be employed whereby the feedstock is treated, e.g. the feedstock is purified to remove sulphur and other potential catalyst poisons prior to being converted into synthesis gas. This treatment can also be conducted after synthesis gas preparation; for example, when coal or biomass is employed.

The reaction to produce ethanol from synthesis gas is generally exothermic. The formation of ethanol is believed to proceed via the formation of methanol for modified methanol synthesis catalysts and cobalt molybdenum sulphide catalysts. However, the production of methanol is equilibrium-limited and thus requires high pressures in order to achieve viable yields.

WO 83/03409 describes a process whereby ethanol is produced by carbonylation of methanol by reaction with CO in the presence of a carbonylation catalyst to form acetic acid which is then converted to an acetate ester followed by hydrogenolysis of the acetate ester formed to give ethanol or a mixture of ethanol and another alcohol which can be separated by distillation. Carbonylation can be effected using a CO/H₂ mixture and hydrogenolysis can similarly be conducted in the presence of CO, leading to the possibility of circulating gas between the carbonylation and hydrogenolysis zones with synthesis gas, preferably a 2:1 H₂:CO molar mixture being used as make up gas.

US 4122110 relates to a process for manufacturing alcohols, particularly linear saturated primary alcohols, by reacting CO with H₂ at a pressure between 2 and 25 MPa and a temperature between 150 and 400 °C, in the presence of a catalyst, characterized in that the catalyst contains at least 4 essential elements: (a) copper (b) cobalt (c) at least one element M selected from chromium, iron, vanadium and manganese, and (d) at least one alkali metal.

US 4831060 relates to the production of mixed alcohols from CO and H₂ gases using a catalyst, with optionally a co-catalyst, wherein the catalyst metals are molybdenum, tungsten or rhenium, and the co-catalyst metals are cobalt, nickel or iron. The catalyst is promoted with a Fischer-Tropsch promoter like an alkali or alkaline earth series metal or a smaller amount of thorium and is further treated by sulphiding. The composition of the mixed alcohols fraction can be selected by selecting the extent of intimate contact among the catalytic components.

Journal of Catalysis, 1988, 114, 90-99 discloses a mechanism of ethanol formation from synthesis gas over CuO/ZnO/Al₂O₃. The formation of ethanol from CO and H₂ over a CuO/ZnO methanol catalyst is studied in a fixed-bed microreactor by measuring the isotopic distribution of the carbon in the product ethanol when isotopically-enriched ¹³C methanol was added to the feed.

WO 2009/077723 discloses a process for the conversion of synthesis gas to ethanol. That process involves the production of ethanol and methanol in an alcohol synthesis unit before their subsequent separation. The separated methanol stream is then conveyed to a carbonylation unit where methanol is converted to acetic acid, which is subsequently conveyed to a separate esterification unit for the formation of methyl or ethyl acetate esters. A key feature of the process is that the product stream from the esterification unit is fed back to the alcohol synthesis unit for conversion to methanol and ethanol. Thus, the synthesis of methanol and ethanol, either from synthesis gas or hydrogenation of alkyl carboxylate esters, occurs in a single alcohol synthesis unit, improving efficiency.

The subsequent production of ethylene by the vapour phase chemical dehydration of ethanol is a well-known chemical reaction which has been operated industrially (see for example Kirk Othmer Encyclopaedia of Chemical Technology (third edition), Volume 9, pages 411 to 413). Traditionally, this reaction has been carried out in the presence of an acid catalyst such as activated alumina or supported phosphoric acid. In recent years attention has turned to finding alternative catalysts having improved performance. This has led to the identification of heteropolyacid catalysts, such as those disclosed in EP1925363, which have the benefit of improved selectivity, productivity and reduced ethane formation following the dehydration of a feedstock comprising ethanol and ethoxyethane for the production of ethylene.

The dehydration of ethanol to form ethylene is a strongly endothermic reaction and therefore involves significant consumption of energy. US 2010/0056831 reports a co-dehydration process of methanol and ethanol as an energy-efficient process for preparing ethylene, from the dehydration of ethanol, together with dimethyl ether, from the dehydration of methanol. Dimethyl ether is known to find application as a fuel, and also as commodity chemical in the pharmaceutical and agrochemical industries. In contrast to the dehydration of ethanol, dehydration of methanol is an exothermic reaction. By coupling the two reactions in a single reactor, the temperature gradient in the reactor may be reduced (thus resulting in a lower maximum temperature) and energy consumption may be reduced.

Methanol itself is also known to be a useful feedstock for the production of olefins, including ethylene, through a methanol-to-olefin (MTO) process (as described in Handbook of Petroleum refining processes third edition, Chapter 15.1 editor R.A. Meyers published by McGraw Hill). The MTO process can be described as the dehydrative coupling of methanol to olefin(s). This mechanism is thought to proceed via a coupling of C₁ fragments generated by the acid catalysed dehydration of methanol, possibly via a methyloxonium intermediate. However the main disadvantage of the MTO process is that a range of olefin(s) are co-produced together with aromatic and alkane by-products, which in turn makes it very difficult and expensive to recover the desired olefin(s) at high purity.

Molecular sieves such as the microporous crystalline zeolite and non-zeolitic catalysts, particularly silicoaluminophosphates (SAPO), are known to promote the conversion of oxygenates by MTO chemistry to hydrocarbon mixtures. Numerous patents describe this process for various types of these catalysts: U.S. Pat. Nos. 3,928,483, 4,025,575,4,252,479 (Chang et al.); 4,496,786 (Santilli et al.); 4,547,616 (Avidan et al.); 4,677,243 (Kaiser); 4,843,183 (Inui); 4,499,314 (Seddon et al.); 4,447,669 (Harmon et al.); 5,095,163 (Barger); 5,191,141 (Barger); 5,126,308 (Barger); 4,973,792 (Lewis); and 4,861,938 (Lewis).

The MTO reaction has a high activation energy, so in order to achieve reasonable rates there is often a need for high temperatures e.g. 300 to 450 °C. However, operating at such high temperatures can lead to major problems such as catalyst deactivation, coking and significant by-product formation. In order to minimize these problems the reactions may be operated at lower temperatures, but this necessitates larger reactors and expensive recycle of intermediates and reactants. Another major disadvantage associated with the MTO method is that the aromatic and alkane by-products that are co-produced together with the olefin(s) and are both difficult and expensive to separate from the desired products e.g. separating ethylene and ethane is an expensive process.

There remains a need for an integrated process for converting carbon monoxide and hydrogen, preferably in the form of synthesis gas, to ethylene that is energy efficient; accommodates recycling of by-products so as to ensure a high yield of the desired ethylene product; and does not necessitate separation of alcohols by distillation. Furthermore, there remains a need for a process which may suitably be operated as a continuous process under steady state conditions.

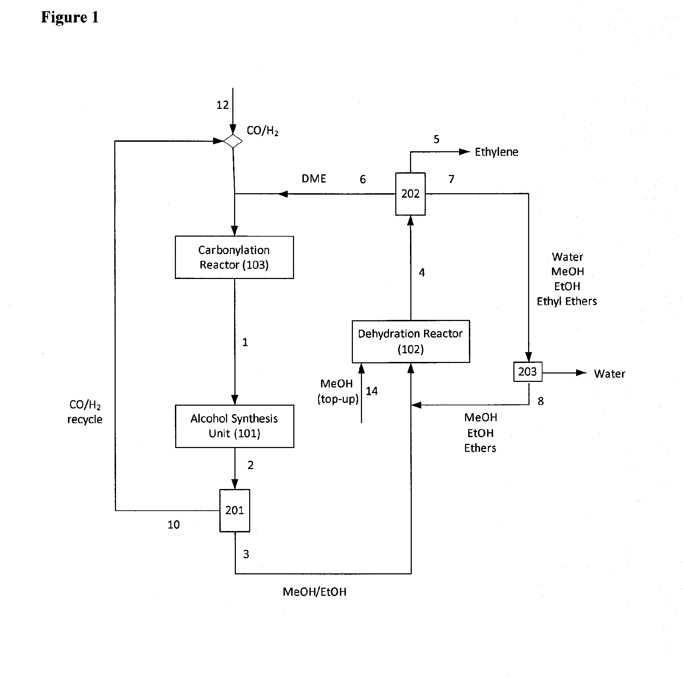

Figure 1 represents an embodiment of an apparatus according to the present invention, wherein the references correspond to those used in the present description.

In a first aspect, the present invention provides a process for the conversion of carbon monoxide and hydrogen to ethylene, said process comprising the following steps:
i) introducing carbon monoxide, hydrogen and methyl acetate into an alcohol synthesis unit to produce a first product stream comprising methanol and ethanol;
ii) separating a methanol and ethanol rich stream and a carbon monoxide and hydrogen rich stream from the first product stream;
iii) introducing at least part of the methanol and ethanol rich stream into a dehydration reactor to produce a second product stream comprising ethylene and dimethyl ether;
iv) separating an ethylene product stream and a dimethyl ether product stream from the second product stream; and
v)introducing carbon monoxide, hydrogen and at least part of the dimethyl ether product stream into a carbonylation reactor to produce a third product stream comprising methyl acetate;
wherein at least a part of the methyl acetate introduced into the alcohol synthesis unit in step i) is recycled from the third product stream produced from the carbonylation reactor in step v); and
wherein the alcohol synthesis unit of step i) comprises a catalyst which is effective to perform hydrogenolysis of methyl acetate and a catalyst which is effective to produce methanol from carbon monoxide and hydrogen, preferably in the form of synthesis gas; and
wherein the carbonylation reactor of step v) comprises a zeolite catalyst effective for said carbonylation reaction.

Furthermore, the present invention also provides an apparatus for the conversion of carbon monoxide and hydrogen to ethylene, said apparatus comprising:
a) an alcohol synthesis unit for producing a first product stream comprising methanol and ethanol from carbon monoxide, hydrogen and methyl acetate;
b) means for separating a methanol and ethanol rich stream and a carbon monoxide and hydrogen rich stream from the first product stream;
c) a dehydration reactor for producing a second product stream comprising dimethyl ether and ethylene from the methanol and ethanol rich stream;
d) means for separating an ethylene product stream and a dimethyl ether product stream from the second product stream;
e) a carbonylation reactor for producing a third product stream comprising methyl acetate from dimethyl ether, carbon monoxide and hydrogen; and
wherein the apparatus is configured such that the carbonylation reactor receives at least part of the separated dimethyl ether product stream; and the alcohol synthesis unit receives at least part of the third product stream from the carbonylation reactor.

For the purposes of the present invention and appending claims the following terms are defined hereinafter:
- The 'dew point temperature' is a threshold temperature, for example, for a given pure component or mixture of components, at a given pressure, if the system temperature is raised to *above* the dew point temperature, the mixture will exist as a dry gas. Likewise *below* the dew point temperature, the mixture will exist as a vapour containing some liquid.
- 'Gas' and/or 'gas phase' are defined as a pure component, or mixture of components, that are above the dew point temperature.
- 'Gas hourly space velocity' (GHSV) is defined as the volume of gas fed per unit volume of catalyst per hour, at standard temperature (0 °C) and pressure (0.101325 MPa).
- 'Liquid hourly space velocity' (LHSV) is defined as the volume of liquid fed per unit volume of catalyst per hour.

Conveniently, in the present invention, multiple reactions corresponding to different stages in the conversion of carbon monoxide and hydrogen to ethylene may be integrated, thereby allowing the use fewer reactors and reducing energy consumption. Alcohol synthesis from i) the reaction of carbon monoxide and hydrogen (Reaction (1) below) and ii) the hydrogenation of methyl acetate (Reaction (4) below) conveniently occur in the same alcohol synthesis unit. Meanwhile methanol and ethanol dehydrations (Reactions (2) and (5) below respectively) also take place in the same dehydration reactor.

CO + 2H₂ → Methanol (1)

2 Methanol → Dimethyl ether + H₂O (2)

Dimethyl ether + CO → Methyl acetate (3)

Methyl acetate + 2H₂ → Ethanol + Methanol (4)

Ethanol → C₂H₄ + H₂O (5)

Moreover, carbonylation of dimethyl ether (Reaction (3) above) in accordance with the present invention, as opposed to, for instance, methanol, has the advantage that it obviates the presence of a distinct esterification unit in order to form acetate esters, from which ethanol may be formed in the alcohol synthesis unit. In prior art processes where it is an object to isolate ethanol as a product (potentially as a feedstock for ethylene production), the methanol by-product from the alcohol synthesis unit is separated. Methanol may be converted to acetic acid in a carbonylation reactor, following which esterification takes place in a separate esterification unit in order to generate acetate esters, which are a suitable feedstock for recycling to the alcohol synthesis unit for the production of ethanol. Carbonylation of dimethyl ether, which is co-produced together with ethylene in the dehydration reactor according to the present invention, corresponds to a more efficient means for generating an ethanol precursor for subsequently recycling to the alcohol synthesis unit.

Consequently, the process of the present invention simultaneously benefits from the energy efficiencies afforded by coupling the dehydration of methanol and ethanol and accommodating different reaction pathways for alcohol formation in a single alcohol synthesis unit, whilst also recycling the product of methanol dehydration (dimethyl ether) for generation of further ethanol, thereby increasing the yield of the ethylene product. Furthermore, carbonylation of dimethyl ether, as opposed to, for example, methanol eliminates the need for esterification units, as described above. Still further, the process of the present invention represents an efficient, integrated process for the production of ethylene from carbon monoxide and hydrogen, preferably in the form of synthesis gas, which process may suitably be operated as a continuous process, under steady state conditions.

According to step i) of the process of the present invention, carbon monoxide, hydrogen and methyl acetate are introduced into an alcohol synthesis unit to produce a first product stream comprising methanol and ethanol.

In one embodiment of the present invention, the carbon monoxide and hydrogen utilised in the process are derived from a synthesis gas feedstock, and is a mixture of carbon oxide(s) and hydrogen, preferably produced from a carbonaceous feedstock. The carbonaceous feedstock is preferably a material such as biomass, plastic, naphtha, refinery bottoms, crude synthesis gas (from underground coal gasification or biomass gasification), smelter off gas, municipal waste, coal bed methane, coal, and/or natural gas, with coal and natural gas being the preferred sources. As will be appreciated by one skilled in the art, a combination of sources can also be used, for example coal and natural gas to advantageously increase the H₂ to carbon ratio.

Methods of preparing synthesis gas, methods for the purification of synthesis gas and/or the carbonaceous feedstock used for the preparation of synthesis gas, processes for adjusting the H₂ to carbon ratio of synthesis gas, and other related processes are well known in the art and may be used prior to using the carbon mononxide and H₂ in the process of the present invention.

In the present invention, the alcohol synthesis unit comprises at least one reactor suitable for producing ethanol and methanol; if more than one reactor is employed in the alcohol synthesis unit, the reactors may be configured in series, in parallel, or in any combination thereof. The reactor(s) used in the alcohol synthesis unit may be a fluidised bed reactor(s) or a fixed bed reactor(s), which can be run with or without external heat exchange equipment e.g. a multi-tubular reactor; or a fluidised bed reactor; or a void reactor.

The alcohol synthesis unit is preferably operated at a temperature of greater than 180 °C, more preferably greater than 190 °C, most preferably greater than 220 °C; and less than 320 °C, preferably less than 300 °C, more preferably less than 290 °C and most preferably less than 280 °C. Examples of preferred ranges of temperature at which the alcohol synthesis unit is operated are from 190 to 300 °C and from 220 to 280 °C. The alcohol synthesis unit is operated at pressure of preferably greater than 50 barg (5.1 MPa) and most preferably greater than 70 barg (7.1 MPa); and preferably less than 100 barg (10.1 MPa) and most preferably less than 90 barg (9.1 MPa). In one particular embodiment, the alcohol synthesis unit is operated such that the reactants and the methanol and ethanol product are in the vapour phase. In fact, since alcohol synthesis is an exothermic reaction, the chosen temperature of operation is governed by a balance of promoting the forward reaction (i.e. by not adversely affecting the equilibrium) and aiding the rate of conversion (i.e. higher productivity).

The GHSV for continuous operation of the alcohol synthesis unit may be in the range 50 to 50,000 h⁻¹, preferably from 1,000 to 30,000 h⁻¹, more preferably from 2,000 to 15,000 h⁻¹ and most preferably from 5,000 to 8,000 h⁻¹.

The methyl acetate introduced into the alcohol synthesis unit preferably has an LHSV of less than 10 h⁻¹, more preferably of less than 5 h⁻¹ and most preferably of less than 3 h⁻¹; for example, a typical LHSV for normal operation is approximately 1.5 h⁻¹.

As described above, an advantage of the process of the present invention is that the synthesis of methanol from synthesis gas and the hydrogenation of methyl acetate to methanol and ethanol (Reactions 1 and 4 above) occurs in the same alcohol synthesis unit.

The catalyst for methanol synthesis from synthesis gas and the catalyst for acetate hydrogenation may be one and the same catalyst; or alternatively more than one catalyst may be employed in the alcohol synthesis unit. The catalyst, or catalysts, may be any catalyst known to those skilled in the art to catalyse the synthesis of methanol from carbon monoxide and hydrogen, preferably in the form of synthesis gas, and those known to those skilled in the art which have been reported to catalyse the hydrogenation of esters to alcohols, in particular methyl acetate. Where there are multiple catalysts employed in the alcohol synthesis unit, these may be provided in separate catalyst beds within the alcohol synthesis unit or in admixture in one or more catalyst beds in the alcohol synthesis unit.

Thus, for the avoidance of doubt, when it is hereinafter referred to as a mixture of a methanol catalyst and a hydrogenation catalyst, it also covers physical blends of the two catalysts and/or separate packed zones of the two catalysts in the same reactor(s).

In one particular embodiment of the present invention, the alcohol synthesis unit comprises a hydrogenation catalyst located downstream of a methanol synthesis catalyst; thus, according to this embodiment of the present invention, the catalyst configuration of the alcohol synthesis unit is such that the stream comprising CO, H₂ and methyl acetate is first reacted in the presence of a methanol synthesis catalyst and subsequently reacted in the presence of a hydrogenation catalyst.

The preferred catalyst for the alcohol synthesis unit can be chosen from one of the two following groups:
i. 'high pressure' zinc catalysts, composed of zinc oxide and optionally a promoter; and
ii. 'low pressure' copper catalysts, composed of zinc oxide, copper oxide, and optionally a promoter.

A preferred methanol synthesis catalyst is a mixture of copper, zinc oxide, and a promoter such as chromia or magnesia.

The aforementioned methanol synthesis catalysts, 'high pressure' zinc catalysts, and 'low pressure' copper catalysts, may optionally be supported on any suitable support known to those skilled in the art. Non-limiting examples of suitable supports for such methanol synthesis catalyst include carbon, silica, titania, clays, aluminas, zinc oxide, zirconia and mixed oxides, with alumina being a particularly suitable support for such methanol synthesis catalysts. Conveniently, however, the aforementioned methanol synthesis catalysts may be used in a non-supported form.

According to a preferred embodiment of the present invention, the preferred catalyst used in the alcohol synthesis unit is either a hydrogenation catalyst or consists of a mixture of the above methanol catalyst together with a hydrogenation catalyst. The hydrogenation catalyst can be selected from the following:
(i) a catalyst comprising of at least one metal from Group VIII of the periodic table (for example iron, ruthenium, osmium, cobalt, rhodium, iridium, nickel, palladium, platinum) and at least one of the metals chosen from rhenium, tungsten and/or molybdenum; and optionally an additional metal, that is capable of alloying with said Group VIII metal;
(ii) a copper-based catalyst (for example a copper chromite or a mixed copper metal oxide based catalyst wherein the second metal can be copper, zinc, zirconium or manganese), and
(iii) mixtures thereof.

According to a preferred embodiment of the present invention, the catalyst(s) used in the alcohol synthesis unit (which may be the aforementioned methanol catalyst and/or the aforementioned hydrogenation catalyst) is a copper based catalyst, most preferably comprising copper and zinc. This copper-based catalyst contains preferably more than 75 wt%, more preferably more than 90 wt% and most preferably more than 95 wt% of copper oxide and zinc oxide.

According to a preferred embodiment of the present invention, the hydrogenation catalyst (which may be mixed with the methanol catalyst) is a copper based catalyst which is a supported catalyst which comprises copper, and preferably promoters, such as cobalt and/or manganese and/or chromium.

All of the aforementioned hydrogenation catalysts may advantageously be supported on any suitable support known to those skilled in the art; non-limiting examples of such supports include carbon, silica, titania, clays, aluminas, zinc oxide, zirconia and mixed oxides. Preferably, when they are used in the form of a supported catalyst, the palladium based catalysts are supported on carbon. Preferably, when they are used in the form of a supported catalyst, the copper based catalysts are supported on zinc oxide.

According to step ii) of the present invention, a stream rich in methanol and ethanol and a stream rich in carbon monoxide and hydrogen are separated from the first product stream of the alcohol synthesis unit. Separation may be achieved by any suitable means known to those skilled in the art. For example, the two streams may be readily separated on the basis of boiling point using, for instance, a condenser and/or phase separator and/or separator column.

For the avoidance of any doubt, where reference is made herein to a methanol and ethanol rich stream obtained from a separation of the first product stream from the alcohol synthesis unit, it is intended to mean a separated stream having an increased combined methanol and ethanol concentration compared to the first product stream from which it is derived. Preferably, methanol and ethanol are the major components of the ethanol and methanol rich stream (i.e. having a combined content of 50 wt.% or greater based on the weight of the stream). Where reference is made herein to a carbon monoxide and a hydrogen rich stream obtained from a separation of the first product stream from the alcohol synthesis unit, it is intended to mean a separated stream having an increased combined carbon monoxide and hydrogen concentration compared to the first product stream from which it is derived. Preferably, carbon monoxide and hydrogen are the major components of the carbon monoxide and hydrogen rich stream (i.e. having a combined content of 50 vol.% or greater based on the volume of the stream).

Preferably, at least a portion of the hydrogen and carbon monoxide rich stream separated from the first product stream of the alcohol synthesis unit is recycled to the alcohol synthesis unit or to the carbonylation reactor.

By-products may be formed within the alcohol synthesis unit, typically by-products that may be produced include water and carbon dioxide, although other by-products such as methane, ethane and other higher alcohols may also be formed in the alcohol synthesis unit. In one particular embodiment of the present invention, following separation of the first product stream according to step ii) of the present invention, the methanol and ethanol rich stream and/or the carbon monoxide and hydrogen rich stream are purified to remove one or more of said by-products before being conveyed to the dehydration reactor or before being recycled, respectively. Purification may be performed by any suitable means known to those skilled in the art, e.g. using a distillation device comprising a sieve tray column, a packed column, a bubble cap column or a combination thereof.

According to step iii) of the process of the invention, at least part of the methanol and ethanol rich stream obtained from the alcohol synthesis unit is introduced into a dehydration reactor to produce a second product stream comprising ethylene and dimethyl ether. Optionally, more than one dehydration reactor may be employed in step iii) of the process of the present invention.

The dehydration of ethanol is believed (Chem. Eng Comm. 1990, 95, 27 to 39) to proceed by either the direct dehydration to olefin(s) and water (Equation 1); or via an ether intermediate (Equations 2 and 3).

Ethanol ⇄ C₂H₄ + H₂O (1)

2 Ethanol ⇄ Diethyl ether + H₂O (2)

Diethyl ether ⇄ C₂H₄ + Ethanol (3)

The direct conversion of the ether to two moles of olefin and water has also been reported (Chem. Eng. Res. and Design 1984, 62, 81 to 91). All of the reactions shown above are typically catalysed by Lewis and/or Brønsted acids. Equation 1 shows the endothermic direct elimination of ethanol to ethylene and water; competing with Equation 1 are Equations 2 and 3 i.e. the exothermic etherification reaction (Equation 2), and the endothermic elimination of ethoxyethane to produce ethylene and ethanol (Equation 3). However, the dehydration reaction of ethanol to ethylene is overall said to be strongly endothermic. In contrast, the dehydration of methanol to dimethyl ether is known to be exothermic (Equation 4).

2 Methanol ⇄ Dimethyl ether + H₂O (4)

Advantageously, heat energy released by the reaction of dehydrating methanol may be taken up for the dehydration reaction of ethanol. Substantial energy savings may thus be made by co-producing dimethyl ether and ethylene from methanol and ethanol respectively in a single dehydration reactor, compared with processes where methanol and ethanol dehydration is decoupled. Coupling the dehydration of methanol and ethanol may also have the advantage that reactor enlargement is more readily achievable, since a single stage fixed bed reactor may be used effectively in favour of, for instance, a multistage fixed bed reactor. A multistage fixed bed reactor is often required in order to have a heat source at multiple positions within the reactor; such a set-up is obviated when heat transfer takes place within the reactor between the different dehydration reactions.

The dehydration reactor is preferably operated at a temperature of greater than 160 °C, more preferably greater than 180 °C, most preferably greater than 200 °C; and less than 350 °C, preferably less than 300 °C, more preferably less than 250 °C and most preferably less than 220 °C. Examples of preferred ranges of temperature at which the dehydration reactor is operated are from 180 to 250 °C, or from 200 to 220 °C. In fact, since ethanol dehydration is an endothermic reaction and methanol dehydration is an exothermic reaction, the chosen temperature of operation is governed by a balance of promoting ethanol and methanol dehydration (i.e. by not adversely affecting the equilibrium) and aiding the rate of conversion (i.e. higher productivity).

The dehydration reactor is operated at pressure of preferably greater than 0.5 barg (0.15 MPa), more preferably greater than 1 barg (0.20 MPa) and most preferably greater than 15 barg (1.60 MPa); and preferably less than 50 barg (5.1 MPa), more preferably less than 30 barg (3.1 MPa) and most preferably less than 25 barg (2.6 MPa).

The GHSV for continuous operation of the dehydration reactor may be in the range 50 to 50,000 h⁻¹, preferably from 1,000 to 30,000 h⁻¹, more preferably from 2,000 to 15,000 h⁻¹ and most preferably from 5,000 to 8,000 h⁻¹.

The molar ratio of methanol : ethanol of the total feed to the dehydration reactor is preferably maintained in the range of from 1 : 1 to 3:1, more preferably from 1.8 : 1 to 2.2 : 1, yet more preferably 1.9 : 1 to 2.1 : 1. Most preferably, the molar ratio of methanol : ethanol of the total feed to the dehydration reactor is maintained at about 2:1.

In an embodiment, the process of the invention further comprises introducing additional methanol into the dehydration reactor from a source which is separate to that of the ethanol and methanol rich stream. When the process of the invention is operated as a continuous process, in order to obtain steady state conditions, it is preferred that methanol is present at higher concentration than ethanol. According to Reactions (2) and (3) above, for every one mole of DME that is recycled, one mole of ethanol may be formed following carbonylation and hydrogenation reactions. However, two moles of methanol are required in order to prepare a single mole of DME. Consequently, in order to operate as a continuous process under steady state mass balance, it is desirable to have a methanol to ethanol ratio in the dehydration reactor of about 2:1.

The catalyst for dehydration of ethanol and the catalyst for dehydration of methanol may be one and the same catalyst; or more than one catalyst may be employed in the dehydration reactor. The catalyst or catalysts may include those which have been reported before to catalyse the dehydration of ethanol to ethylene and/or catalyse the dehydration of methanol to dimethyl ether. Such catalysts include solid-acid catalysts.

Preferred classes of solid-acid catalyst for use in the dehydration reactor are aluminas, heteropolyacids, crystalline alumina silicates (e.g. zeolites), and silicoaluminophosphates (SAPO).

Preferred alumina catalysts for use in the dehydration reactor comprise γ-Al₂O₃.

The term "heteropolyacid" (also referred to herein as "HPA"), as used herein is deemed to include *inter alia*; alkali, alkali earth, ammonium, free acids, bulky cation salts, and/or metal salts (where the salts may be either full or partial salts) of heteropolyacids. Hence, the heteropolyacids that may be used in conjunction with the present invention are complex, high molecular weight anions comprising oxygen-linked polyvalent metal atoms. Typically, each anion comprises 12-18, oxygen-linked polyvalent metal atoms. The polyvalent metal atoms, known as peripheral atoms, surround one or more central atoms in a symmetrical manner. The peripheral atoms may be one or more of molybdenum, tungsten, vanadium, niobium, tantalum, or any other polyvalent metal. The central atoms are preferably silicon or phosphorus, but may alternatively comprise any one of a large variety of atoms from Groups I- VIII in the Periodic Table of elements. These include copper, beryllium, zinc, cobalt, nickel, boron, aluminium, gallium, iron, cerium, arsenic, antimony, bismuth, chromium, rhodium, silicon, germanium, tin, titanium, zirconium, vanadium, sulphur, tellurium, manganese, platinum, thorium, hafnium, cerium, arsenic, vanadium, antimony ions, tellurium and iodine. Suitable heteropolyacids include Keggin, Wells-Dawson and Anderson-Evans-Perloff heteropolyacids. Specific examples of suitable heteropolyacids are as follows:

| | |
|---|---|
| 18-tungstophosphoric acid | - H₆[P₂W₁₈O₆₂].xH₂O |
| 12-tungstophosphoric acid | - H₃[PW₁₂O₄₀].xH₂O |
| 12-tungstosilicic acid | - H₄[SiW₁₂O₄₀].xH₂O |
| Cesium hydrogen tungstosilicate | - Cs₃H[SiW₁₂O₄₀].xH₂O |

and the free acid or partial salts of the following heteropolyacids acids:

| | |
|---|---|
| Monopotassium tungstophosphate | - KH₅[P₂W₁₈O₆₂].xH₂O |
| Monosodium 12-tungstosilicic acid | - NaK₃[SiW₁₂O₄₀]·xH₂O |
| Potassium tungstophosphate | - K₆[P₂W₁₈O₆₂].xH₂O |
| Ammonium molybdodiphosphate | - (NH₄)₆ [P₂Mo₁₈O₆₂].xH₂O |
| Potassium molybdodivanado phosphate | - K₅[PMoV₂O₄₀].xH₂O |

In addition, mixtures of different heteropolyacids and salts can be employed. Preferably, in the embodiments wherein a heteropolyacid catalyst is used in the dehydration step iii), the heteropolyacid is selected from one or more heteropolyacids that are based on the Keggin or Wells-Dawson structures; more preferably the heteropolyacid is selected from one or more of the following: heteropolytungstic acid (such as silicotungstic acid and phosphotungstic acid), silicomolybdic acid and phosphomolybdic acid. Most preferably, the heteropolyacid is any one or more silicotungstic acid, for example 12-tungstosilicic acid (H₄[SiW₁₂O₄₀].xH₂O).

Preferably, heteropolyacids employed in the dehydration step iii) of the process of the present invention may have molecular weights of more than 700 and less than 8500, preferably more than 2800 and less than 6000. Such heteropolyacids also include dimeric complexes.

Preferably, the heteropolyacids employed in the dehydration step iii) of the process of the present invention are supported. The amount of heteropolyacid on the support is suitably in the range of 10 wt % to 80 wt % and preferably 20 wt % to 50 wt % based on the total weight of the heteropolyacid and the support. The preferred catalytic loading of heteropolyacid is 150 to 600g heteropolyacid / kg Catalyst.

It should be noted that the polyvalent oxidation states and hydration states of the heteropolyacids stated previously and as represented in the typical formulae of some specific compounds only apply to the fresh acid before it is loaded onto the support, and especially before it is subjected to the dehydration process conditions. The degree of hydration of the heteropolyacid may affect the acidity of the supported catalyst and hence its activity and selectivity. Thus, either or both of these actions of impregnation and dehydration process may change the hydration and oxidation state of the metals in the heteropolyacids, i.e. the actual catalytic species used, under the process conditions given, may not yield the hydration/oxidation states of the metals in the heteropolyacids used to impregnate the support. Naturally therefore it is to be expected that such hydration and oxidation states may also be different in the spent catalysts after reaction.

Where a supported heteropolyacid catalyst is used in conjunction with the process of the present invention, it may be a fresh catalyst or a previously used catalyst. Thus, in one embodiment, at least a portion of the supported heteropolyacid catalyst has previously been employed in a process for the preparation of an ethylene and/or dimethyl ether from a feed comprising ethanol and/or methanol respectively. For example, at least a portion of the supported heteropolyacid catalyst may derive from an extract of heteropolyacid from a previously used catalyst i.e. from a partially deactivated material.

According to a further embodiment of the present invention, a heteropolytungstic acid supported catalyst having the following characteristic is employed in the dehydration step according to step iii):
PV > 0.6 - 0.3 x [HPA loading/Surface Area of Catalyst]
wherein PV is the pore volume of the dried supported heteropolytungstic acid catalyst (measured in ml/g catalyst); HPA loading is the amount of heteropolyacid present in the dried supported heteropolyacid catalyst (measured in micro moles per gram of catalyst) and Surface Area of Catalyst is the surface area of the dried supported heteropolytungstic acid catalyst (measured in m² per gram of catalyst).

Suitable catalyst supports may be in a powder form or alternatively may be in a granular form, or in a pelletised form, a spherical form or as extrudates (including shaped particles) and include, but are not limited to, clays, bentonite, diatomaceous earth, titania, activated carbon, aluminosilicates e.g. montmorillonite, alumina, silica-alumina, silica-titania cogels, silica-zirconia cogels, carbon coated alumina, zeolites, zinc oxide, flame pyrolysed oxides.

The average pore radius (prior to loading with the heteropolyacid) of the support is 10 to 500Å, preferably 30 to 175Å, more preferably 50 to 150 Å and most preferably 60 to 120Å. The BET surface area is preferably between 50 and 600 m²/g and is most preferably between 150 and 400 m²/g.

Another preferred class of solid-acid catalyst which may suitably be used in the dehydration reaction according to step iii) of the process of the present invention is a zeolite, preferably in the form of the acid (H-form); a non-limiting example of a suitable zeolite includes the zeolites having the faujasite (FAU) framework type, such as faujasite.

According to step iv) of the process of the invention, an ethylene product stream and a dimethyl ether product stream are separated from the second product stream obtained from the dehydration reactor. Separation may be performed by any suitable means known to those skilled in the art, e.g. a flash separation device, a distillation device comprising a sieve tray column, a packed column, a bubble cap column or a combination thereof. Alternatively or in addition, the separation means may comprise a membrane component.

The ethylene product stream may undergo further purification following its separation from the second product stream from the dehydration reaction, prior to storage or use as a chemical feedstock. Purification may be performed by any suitable means known to those skilled in the art, e.g. a sieve tray column, a packed column, a bubble cap column or a combination thereof.

In addition to DME, ethylene and water, the dehydration reaction may also form ethyl ethers such as diethyl ether and methyl ethyl ether. In a preferred embodiment, separation step iv) further comprises a step of obtaining a recycle stream comprising water, unreacted alcohols (e.g. methanol and/or ethanol) and any ethyl ethers, such as DEE and MEE, that may have formed, and introducing the recycle stream into the dehydration reactor. Any DEE and MEE that may be formed in the dehydration reaction may be further reacted in the dehydration reactor to produce ethylene product and DME, as illustrated below (Reactions 6 and 7):

(CH₃CH₂)₂O → 2 CH₂CH₂ + H₂O (6)

2 CH₃CH₂OCH₃ → 2 CH₂CH₂ + CH₃OCH₃ + H₂O (7)

According to a preferred embodiment, water is removed from the above-mentioned recycle stream by any suitable means known to the skilled person, for example a condenser and/or phase separator, prior to its introduction into the dehydration reactor.

According to another preferred embodiment of this aspect of the present invention, following separation of the first product stream according to step ii) of the present invention, the methanol and ethanol rich stream and/or the carbon monoxide and hydrogen rich stream are purified to remove one or more of said by-products before being conveyed to the dehydration reactor or before being recycled, respectively. Purification may be performed by any suitable means known to those skilled in the art, e.g. a sieve tray column, a packed column, a bubble cap column or a combination thereof.

According to step v) of the process of the present invention, at least part of the dimethyl ether product stream, together with a stream comprising carbon monoxide and hydrogen, is introduced into a carbonylation reactor to produce a third product stream comprising methyl acetate. The carbonylation step v) is performed in the presence of a zeolite catalyst effective for said carbonylation.

The dimethyl ether component of the feed for carbonylation step v) of the process of the present invention is derived at least in part from dimethyl ether produced from the dehydration of methanol in step iii) of the process of the invention. Preferably, the dimethyl ether component of the feed for carbonylation step v) is derived exclusively from dimethyl ether produced from the dehydration of methanol in step iii) of the process of the invention.

The feed to the carbonylation reactor may also comprise small amounts of other components arising from an imperfect separation of components contained in the dehydration product stream in step iv) of the process of the present invention. Thus, the feed for the carbonylation reactor may comprise small amounts of methyl ethyl ether (MEE), diethyl ether (DEE), methanol, ethanol and/or water, provided that the amount of these components is not so great as to inhibit the carbonylation of dimethyl ether to methyl acetate.

Suitably, dimethyl ether is present in the feed at a concentration in the range 0.1 to 20 mol%, based on the total feed (including recycles).

The carbon monoxide may be substantially pure carbon monoxide, for example, carbon monoxide typically provided by suppliers of industrial gases, or it may contain impurities that do not interfere with the conversion of the dimethyl ether to methyl acetate, such as nitrogen, helium, argon, methane and/or carbon dioxide. In the embodiments of the present invention wherein the feed to the carbonylation reactor derives at least in part from the carbon monoxide and hydrogen rich stream separated from the first product stream in step ii), the feed to the carbonylation reactor may also comprise some methanol, ethanol, methyl acetate and ethyl acetate which may be present in said carbon monoxide and hydrogen rich stream being recycled.

Carbonylation step v) of the process of the present invention is carried out in the presence of hydrogen. The hydrogen may be fed as a separate stream to the carbonylation reactor or it may be fed in combination with, for example carbon monoxide. Thus, the feed for the carbonylation reactor may comprise synthesis gas. The feed to the carbonylation reactor may also comprise carbon monoxide and hydrogen derived from the carbon monoxide and hydrogen rich stream which is separated from the first product stream in step ii) of the process of the present invention and recycled to the carbonylation reactor.

Suitably, the molar ratio of carbon monoxide : hydrogen in the feed to the carbonylation reactor may be in the range 1 : 3 to 15 : 1. The molar ratio of (carbon monoxide + hydrogen) : (dimethyl ether + methyl acetate) is suitably in the range of 100:1 to 5:1, preferably 50 : 1 to 10 : 1.

In a preferred embodiment, the carbonlyation step v) of the process of the present invention is performed under substantially anhydrous conditions. Preferably, the process of the present invention further comprises a step of drying at least part of the dimethyl ether product stream obtained from the dehydration reactor prior to its introduction into the carbonylation reactor. In this embodiment, the means for drying the dimethyl ether product stream obtained from the dehydration reactor is not limited and any suitable method of drying the dimethyl ether product stream may be used; methods of drying dimethyl ether are well known in the art.

The zeolite catalyst may be any zeolite which is effective to catalyse the carbonylation of dimethyl ether in the presence of carbon monoxide and hydrogen to produce methyl acetate. Examples of zeolites suitable for use in carbonylation step of the present invention include zeolites of framework type MOR, for example mordenite, FER, such as ferrierite, OFF, for example, offretite and GME, for example gmelinite. Thus, in one particular meboedioment of the process of the present invention, the zeolite catalyst present in the carbonylation reactor is selected from the group consisting of zeolites of framework type MOR, FER, OFF and GME.

In a preferred embodiment, the zeolite catalyst is dried prior to use. The zeolite may be dried, for example by heating to a temperature of 400 to 500 °C.

The carbonylation step is preferably carried out under substantially anhydrous conditions, i.e. in the substantial absence of water. The carbonylation of dimethyl ether to methyl acetate does not generate water in-situ. Water has been found to inhibit the carbonylation of dimethyl ether to form methyl acetate. Thus, in carbonylation step v) of the process of the present invention, water is kept as low as is feasible. To accomplish this, the dimethyl ether and carbon monoxide reactants (and catalyst) are preferably dried prior to introduction into the process. However, small amounts of water may be tolerated without adversely affecting the formation of methyl acetate. Suitably, the dimethyl ether may contain, 2.5 wt% or less.

Preferably, the total combined amount of water and water equivalents present in the carbonylation reactor is less than 500 ppm relative to the amount of dimethyl ether. As is understood by the skilled person, alcohols and ethers present in the carbonylation reactor can be dehydrated over the zeolite catalyst to yield water in addition to the olefin and ether products. Di-ethyl ether, methanol and ethanol each constitute one equivalent of water, whereas methyl-ethyl ether constitutes half an equivalent of water.

The carbonylation step v) of the process of the present invention may suitably be carried out at a temperature in the range of 150 to 350°C.

The carbonylation step v) of the process of the present invention may suitably be carried out at a total pressure in the range 1 to 100 barg (0.20 MPa to 10.1 MPa). The hydrogen partial pressure is suitably in the range 0.1 to 50 barg (0.11 MPa to 5.1 MPa). The carbon monoxide partial pressure should be sufficient to permit the production of methyl acetate product but is suitably in the range 0.1 to 50 barg (0.11 MPa to 5.1 MPa). The Gas Hourly Space Velocity (GHSV) is suitably in the range 500 to 40,000 h⁻¹, such as 2000 to 10,000 h⁻¹, for example 4000 to 6000 h⁻¹.

The carbonylation step v) of the process of the present invention is suitably carried out by passing dimethyl ether vapour, hydrogen gas and carbon monoxide gas through a fixed or fluidised bed of the zeolite catalyst maintained at a desired temperature.

The primary product of the carbonylation step v) is methyl acetate but small amounts of acetic acid may also be produced. The methyl acetate produced by the carbonylation step v) can be removed in the form of a vapour and thereafter condensed to a liquid.

At least a part of the methyl acetate introduced into the alcohol synthesis unit in step i) of the process of the present invention is recycle from the third product stream produced from the carbonylation reactor in step v).

In a preferred embodiment, methyl acetate which is introduced into the alcohol synthesis unit in step i) of the process of the present invention is obtained entirely from recycle of the third product stream produced from the carbonylation reactor in step v). This embodiment of the present invention is particularly advantageous where the process is operated as a continuous process. Preferably, the third product stream obtained from the carbonylation reactor comprising methyl acetate is conveyed directly from the carbonylation reactor to the alcohol synthesis unit, without any intermediate processing steps.

Alternatively the third product stream obtained from the carbonylation reactor may undergo a partial separation and part of the stream may be recycled to the carbonylation reactor. Alternatively or additionally, carbon monoxide and hydrogen which is fed to the carbonylation reactor in step v) may derive at least in part from the carbon monoxide and hydrogen rich stream separated from the first product stream in step ii).

In one embodiment, the carbonylation reactor may take the form of a catalyst bed stack in the alcohol synthesis unit. This embodiment may have advantages in terms of space considerations and there may be further energy benefits in conducting multiple reactions within the same unit, operated under the same physical conditions of, for instance temperature and pressure. Alternatively, the carbonylation reactor may be separate from the alcohol synthesis unit, which has the advantage that the hydrogenation and carbonylation reactions may each be run at their optimal conditions of temperature and pressure.

It should be noted that whilst all of the aforementioned temperature and pressure operating conditions form preferred embodiments of the present invention, they are not, by any means, intended to be limiting, and the present invention hereby includes any other pressure and temperature operating conditions that achieve the same effect.

The invention will now be described with reference to a preferred embodiment of the invention and with the aid of the accompanying Figure 1.

Figure 1 shows an apparatus in accordance with the invention. The apparatus comprises an alcohol synthesis unit (101) comprising a catalyst which is effective to perform hydrogenolysis of methyl acetate and a catalyst which is effective to produce methanol from carbon monoxide and hydrogen, preferably in the form of synthesis gas. The alcohol synthesis unit (101) may be any reactor that is suitable for producing methanol and ethanol. For example, the alcohol synthesis unit (101) may be a fluidised bed reactor or a fixed bed reactor, which can be run with or without external heat exchange equipment e.g. a multi-tubular reactor; or a fluidised bed reactor; or a void reactor.

The feed stream (1) to the alcohol synthesis unit (101) comprises carbon monoxide, hydrogen and methyl acetate. As illustrated in Figure 1, at least a part of the methyl acetate supplied to the alcohol synthesis unit (101) is produced by a carbonylation reactor (103), described in further detail below, which also forms part of the apparatus.

The stream (2) which exits the alcohol synthesis unit (101), referred to hereinbefore as the first product stream, comprises, *inter alia*, methanol, ethanol and unreacted carbon monoxide and hydrogen. The first product stream (2) is conveyed to a separation means (201), where a carbon monoxide and hydrogen rich stream (10) and a methanol and ethanol rich stream (3) are separated therefrom. Separation means (201) is any suitable means of which the skilled person is aware which can be used to separate carbon monoxide and hydrogen (i.e. components which are gaseous at room temperature at atmospheric pressure) from methanol and ethanol (i.e. components which are liquid at room temperature at atmospheric pressure). Separation means (201) may, for instance, be a condenser and/or phase separator. Thus, carbon monoxide and hydrogen gases may be separated from methanol and ethanol liquids by a simple gas-liquid phase separation, in order to produce separated streams (10, 3). Carbon monoxide and hydrogen rich stream (10) may be recycled to the carbonylation reactor (103), as shown Figure 1. Alternatively or additionally, all or a part of the carbon monoxide and hydrogen rich stream (10) may be recycled back to the alcohol synthesis unit (101).

Methanol and ethanol rich stream (3) is conveyed to the dehydration reactor (102). The dehydration reactor (102) comprises a catalyst(s) to catalyse the dehydration of ethanol to ethylene and to catalyse the dehydration of methanol to dimethyl ether, such as solid-acid catalysts preferably selected from heteropolyacids, aluminas, crystalline alumina silicates (e.g. zeolites), and silicoaluminophosphates (SAPO). The stream (4) which exits the dehydration reactor (102), referred to hereinbefore as the second product stream, comprises ethylene and dimethyl ether, as well as, *inter alia*, water, ethyl ethers (such as DEE and MEE) and unreacted alcohol (i.e. methanol and ethanol). The second product stream (4) is conveyed to separating means (202), where an ethylene product stream (5) and dimethyl ether product stream (6) are separated therefrom. Separation means (202) may comprise, for instance, a distillation device incorporating a sieve tray column, a packed column, a bubble cap column or a combination thereof. Alternatively or in addition, the separation means (202) may comprise a membrane component.

A recycle stream (7) for the dehydration reactor may also be obtained from the separation of the second product stream (4) as shown in Figure 1, comprising, *inter alia*, water, methanol, ethanol and ethyl ethers (e.g. MEE and DEE). Before recycle stream (7) is conveyed back to the dehydration reactor (102), water is preferably removed by a suitable separation means (203). Separation means (203) is any suitable means of which the skilled person is aware which can be used to separate water from lighter organic solvents, such as alcohols and ethyl ethers. Thus, the separation means (203) may comprise a distillation device incorporating a sieve tray column, a packed column, a bubble cap column or a combination thereof. Alternatively or additionally, the separation means (203) may comprise a membrane component. The stream (8), comprising predominantly methanol, ethanol and ethyl ethers, is conveyed to the dehydration reactor (102) for recycle.

Optionally, a separate methanol top-up stream (14) may also be provided in order to increase the level of methanol. As discussed hereinbefore, it is particularly desirable to operate the process of the invention such that the molar ratio of methanol: ethanol is about 2:1.

As illustrated, the apparatus of Figure 1 is configured such that at least a part of the dimethyl ether product stream (6) separated from the second product stream of the dehydration reactor (102) is conveyed to the carbonylation reactor (103). In addition, carbon monoxide and hydrogen from a carbon monoxide and hydrogen feed stream (12) and optionally also from recycle of carbon monoxide and hydrogen from the carbonylation reactor (103) (not shown) and/or recycle of the separated carbon monoxide and hydrogen rich stream (10). Prior to introduction of at least part of the dimethyl ether stream (6) into the carbonylation reactor (103), the stream (6) may optionally be dried to reduce or remove any residual water.

The carbonylation reactor (103) comprises a zeolite catalyst suitable for converting dimethyl ether to methyl acetate in the presence of carbon monoxide and hydrogen. The stream (1) which exits the carbonylation reactor (103), referred to hereinbefore as the third product stream, comprises methyl acetate, in addition to carbon monoxide and hydrogen.

As illustrated, the apparatus of Figure 1 is configured such that at least part of the third product stream (1) is conveyed to the alcohol synthesis unit (101).

As will be appreciated from Figure 1, which illustrates a preferred embodiment, the apparatus of the invention is configured so as to minimise the number of reactors which are necessary for the conversion of carbon monoxide and hydrogen, preferably in the form of synthesis gas, to ethylene, whilst incorporating recycle of the products of methanol dehydration, so as to increase the yield of ethylene. In addition, it will be appreciated from the appended figure that the process of the present invention may advantageously be operated as a continuous process.

### Reference Example

### Assessing selectivity for co-dehydration of methanol and ethanol

An assessment of the selectivity for the co-dehydration reaction of methanol and ethanol was made. Dehydration of a stream, corresponding to the first product stream (3) as defined hereinbefore and with reference to Figure 1, comprising a mixture of methanol : ethanol: nitrogen in a molar ratio of 1.4 : 1 : 3.1 was assessed on the basis of a dehydration in a dehydration reactor run at a temperature of 280 °C and at ambient pressure, over a zeolite-Y catalyst (faujasite) in the proton form for 7 hours at a GHSV of 1500 h⁻¹. The yield of dimethyl ether (DME) based on the moles of methanol in the feed was 30%, whilst the yield of ethylene based on the moles of ethanol in the feed was 73%.

Table 1 below shows the composition of the effluent stream from the dehydration reactor, corresponding to the second product stream (4) as described hereinbefore and with reference to Figure 1, as determined by gas chromatography.

**Table 1**

| **Component** | **mol%** |
|---|---|
| Methane | 0.02 |
| Ethane | 0.05 |
| Ethylene | 29.91 |
| Propane | 0.02 |
| Propylene | 0.12 |
| Iso-Butane | 0.10 |
| n-Butane | 0.01 |
| *trans-*2-Butene | 0.03 |
| 1-Butene | 0.01 |
| Iso-Butylene | 0.04 |
| Cis-2-Butene | 0.02 |
| i-Pentane | 0.04 |
| n-Pentane | 0.06 |
| Unknown | 0.00 |
| Hydrogen | 0.00 |
| CO | 0.00 |
| C6+ | 0.09 |
| DME | 8.93 |
| Methanol | 12.19 |
| Ethanol | 3.18 |
| Unknown at 3.16 mins | 0.06 |
| Unknown at 3.86 mins | 0.08 |
| Water | 49.24 |
| C9-C12 Aromatics | 0.00 |

As can be seen from Table 1, the principal components of the product stream from the dehydration reactor are ethylene, dimethyl ether, methanol, ethanol and water. This example shows that co-dehydration of methanol and ethanol is possible in a single reactor. In particular, the yield of the desired ethylene product in ethanol dehydration is high at 73%. This example also demonstrates that the principal components of the effluent stream, aside from the desired ethylene product, are materials that are either committed to further processing (e.g. DME for use in the carbonylation reaction) or otherwise recycled (e.g. ethanol and methanol) in order to increase ethylene production either directly (i.e. by dehydration of ethanol) or indirectly by preparation of further dimethyl ether for subsequent conversion to an ethanol precursor (methyl acetate).

## Claims

1. A process for the conversion of carbon monoxide and hydrogen to ethylene, said process comprising the following steps:
i) introducing carbon monoxide, hydrogen and methyl acetate into an alcohol synthesis unit to produce a first product stream comprising methanol and ethanol;
ii) separating a methanol and ethanol rich stream and a carbon monoxide and hydrogen rich stream from the first product stream;
iii) introducing at least part of the methanol and ethanol rich stream into a dehydration reactor to produce a second product stream comprising ethylene and dimethyl ether;
iv) separating an ethylene product stream and a dimethyl ether product stream from the second product stream; and
v) introducing carbon monoxide, hydrogen and at least part of the dimethyl ether product stream into a carbonylation reactor to produce a third product stream comprising methyl acetate;
wherein at least a part of the methyl acetate introduced into the alcohol synthesis unit in step i) is recycled from the third product stream produced from the carbonylation reactor in step v); and
wherein the alcohol synthesis unit of step i) comprises a catalyst which is effective to perform hydrogenolysis of methyl acetate and a catalyst which is effective to produce methanol from carbon monoxide and hydrogen, preferably in the form of synthesis gas; and
wherein the carbonylation reactor of step v) comprises a zeolite catalyst effective for said carbonylation reaction.

2. A process according to Claim 1 wherein the catalyst which is effective to perform hydrogenolysis of methyl acetate and the catalyst which is effective to produce methanol from carbon monoxide and hydrogen is the same catalyst.

3. A process according to claim 1 or claim 2, wherein methyl acetate introduced into the alcohol synthesis unit is obtained entirely from recycle of the third product stream.

4. A process according to any one of the preceding claims, wherein carbon monoxide and hydrogen which is fed to the carbonylation reactor in step v) derives at least in part from the carbon monoxide and hydrogen rich stream separated from the first product stream in step ii).

5. A process according to any one of the preceding claims, further comprising introducing additional methanol into the dehydration reactor from a source which is separate to that of the ethanol and methanol rich stream.

6. A process according to any one of the preceding claims, wherein the methanol: ethanol ratio of the total feed to the dehydration reactor is maintained at a ratio of from 1.8 : 1 to 2.2 : 1; preferably 1.9 : 1 to 2.1 : 1; more preferably at 2:1.

7. A process according to any one of the preceding claims, wherein the catalyst(s) in the dehydration reactor is selected from the group consisting of heteropolyacids, aluminas, crystalline alumina silicates (e.g. zeolites), and silicoaluminophosphates (SAPO).

8. A process according to any one of the preceding claims, further comprising a step of drying the at least part of the dimethyl ether product stream prior to its introduction into the carbonylation reaction.

9. A process according to any one of the preceding claims, wherein the separation step iv) further comprises obtaining a recycle stream comprising unreacted alcohols and ethyl ethers (methyl ethyl ether and diethyl ether) and introducing the recycle stream into the dehydration reactor.

10. A process according to any one of the preceding claims, wherein the zeolite catalyst present in the carbonylation reactor is selected from the group consisting of zeolites of framework type MOR, FER, OFF and GME.

11. A process according to any one of the preceding claims, wherein the carbonylation step v) is performed under substantially anhydrous conditions.

12. An apparatus for the conversion of carbon monoxide and hydrogen to ethylene, said apparatus comprising:
a) an alcohol synthesis unit for producing a first product stream comprising methanol and ethanol from a feed comprising carbon monoxide, hydrogen and methyl acetate;
b) means for separating a methanol and ethanol rich stream and a carbon monoxide and hydrogen rich stream from the first product stream;
c) a dehydration reactor for producing a second product stream comprising dimethyl ether and ethylene from the methanol and ethanol rich stream;
d) means for separating an ethylene product stream and a dimethyl ether product stream from the second product stream;
e) a carbonylation reactor for producing a third product stream comprising methyl acetate from a feed comprising dimethyl ether, carbon monoxide and hydrogen; and
wherein the apparatus is configured such that the carbonylation reactor receives at least part of the separated dimethyl ether product stream produced in the dehydration reactor; and the alcohol synthesis unit receives at least part of the third product stream.

13. An apparatus according to Claim 12, which is configured such that the carbon monoxide and hydrogen rich stream separated by separating means b) may be supplied at least in part to the carbonylation reactor.

14. An apparatus according to Claim 12 or Claim 13, additionally comprising means for drying at least part of the separated dimethyl ether product stream prior to its introduction into the carbonylation reaction.

15. An apparatus according to any one of Claims 12 to 14, wherein the separation by separating means d) also obtains a recycle stream comprising unreacted alcohols and ethyl ethers and water from the second product stream and the apparatus is configured such that said recycle stream may be recycled to the dehydration reactor.
